(19) Europäisches Patentamt
European Patent Office
Office européen des brevets

(11) **EP 2 934 408 B1**

(12) **EUROPEAN PATENT SPECIFICATION**

(45) Date of publication and mention
of the grant of the patent:
**04.04.2018 Bulletin 2018/14**

(21) Application number: **12890399.4**

(22) Date of filing: **20.12.2012**

(51) Int Cl.:
*A61F 13/53* (2006.01)     *A61F 13/472* (2006.01)
*A61F 13/534* (2006.01)

(86) International application number:
**PCT/SE2012/051462**

(87) International publication number:
**WO 2014/098679 (26.06.2014 Gazette 2014/26)**

(54) **ABSORBENT ARTICLE**

SAUGFÄHIGER ARTIKEL

ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**28.10.2015 Bulletin 2015/44**

(73) Proprietor: **SCA Hygiene Products AB
405 03 Göteborg (SE)**

(72) Inventors:
• **ABBAS, Shabira
S-431 69 Mölndal (SE)**
• **BAGGER-SJÖBÄCK, Anna
S-414 63 Göteborg (SE)**
• **BURVALL, Angelica
S-517 37 Bollebygd (SE)**
• **PALMQVIST, Lisa
S-431 49 Mölndal (SE)**
• **SAMUELSSON, Ann
S-437 32 Lindome (SE)**

(74) Representative: **Zacco Sweden AB
P.O. Box 5581
114 85 Stockholm (SE)**

(56) References cited:
EP-A1- 0 336 578        WO-A1-2011/158146
US-A- 5 814 034         US-A1- 2002 156 443
US-A1- 2005 131 371     US-A1- 2005 131 371
US-A1- 2008 103 468     US-A1- 2011 166 540

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL AREA

The invention relates to a folded absorbent product that has at least one fold in the transverse direction of the product, wherein the absorbent product comprises an absorption body.

BACKGROUND

It is desirable that absorbent products such as sanitary napkins, panty liners and incontinence pads are packaged in such a way so they take up as little volume in the packaging as possible. If the product takes up a small volume in the packaging, i.e. during storage and transport, the transport cost is reduced and also the size of the storage space that is required for the absorbent products manufactured. Absorbent products such as sanitary napkins, panty liners and incontinence pads often have one or more folds in the packaging, therefore, for example in the transverse direction of the product. In the manufacture of absorbent products, it usually occurs that the product is compressed, and compression of the product is often carried out after the folding stage but before the packaging stage. Due to such a compression stage, the product becomes thinner and takes up a smaller volume, which makes it possible to place several products in a packaging bag of a certain size, or alternatively the packaging bags can be made smaller but with an unchanged number of products in the packaging.

One problem when using absorbent products that have been folded and compressed prior to packaging is that creases can be formed in the product along the fold. These creases can be troublesome during the use of the products. If the products are folded in their transverse direction, for example, creases are often obtained in the transverse direction of the product, making it possible for liquid to run along the creases and be transported out to the product's edges and create a liquid leakage. Furthermore, creases due to folding of the product in its transverse direction can cause material breaks in the material layer of the absorption body along the fold, which leads to an impaired capacity to spread and distribute the liquid in the absorption body. Such creases risk impairing the liquid-absorbent capacity of the absorption body. Material breaks in the absorption body can also impair the function of the absorption body as a shaping element, and mean that the absorption body crumples.

JP 2010178932 A relates to a folded towel, the towel having one or more continuous channels that each extend along both the longitudinal and the transverse direction of the product, wherein the channels in the transverse direction of the product coincide with the fold line that is formed due to the product having been folded together in the transverse direction of the product during packing. The channels in the product's longitudinal direction assist in guiding the liquid along the channels in the product's longitudinal direction, which counteracts the liquid being guided along the product's transverse fold line, which reduces the risk of lateral leakage. US2005/0131371A1 and US2008/0103468A1 disclose folded absorbent articles.

Another problem linked to creases formed by folding the product together is that the creases can make attachment of the product in the user's briefs difficult. Towels are often attached in the briefs by means of an adhesive on the underside of the backing layer and for the adhesive to be attached to the briefs it is essential that the towel is relatively flat upon attachment, i.e. does not have creases and/or folds, which can mean that the full extension of the adhesive does not stick down to the briefs.

Furthermore, it is essential that the absorbent product is experienced as reliable and that the user has a feeling of reliability in that the product shall function well.

DESCRIPTION OF THE INVENTION

The problem of achieving a product that has high leakage security, is soft, and can be packed in such a way that the transport cost and storage space is minimised has essentially been avoided by the present invention.

A product made in accordance with the invention is distinguished principally in that the absorption body comprises a liquid-receiving, flexible foam layer and a liquid-absorbent fibrous layer, wherein the liquid-receiving, flexible foam layer has a total surface in the plane extension of the product that at least covers the entire surface of the liquid-absorbent, fibrous layer in the plane extension, wherein the liquid-receiving, flexible foam layer has a bending stiffness that is lower than 0.30 newtons measured according to the modified Circular Bend Procedure method, and that the liquid-absorbent, fibrous layer has a bending stiffness that is higher than 2.0 newtons, measured according to the modified Circular Bend Procedure method. An advantage of the liquid-absorbent layer having a bending stiffness that is greater than 2.0 newtons is that it creates a relatively stiff and stable product, which means that the liquid-absorbent layer also functions as a stiffening element. The bending stiffness means that the product does not become so limp and that it cannot so readily shrink together and create undesired creases in the crotch area. In order to also achieve a product that is soft, flexible and comfortable to wear, it has proved to be important that the liquid-receiving layer is a flexible foam material. Flexible foam materials can spring back, i.e. return to substantially their original shape in all directions after having been exposed to external loading. The flexible foam material thus has a resilient effect and is not appreciably influenced, therefore, by the force that the material is exposed to upon folding. The flexible foam material can also dampen the force that the product's underlying fibrous liquid-absorbent layer is exposed to upon folding. The foam material thus has a shock-absorbing effect in relation to the fibrous, liquid-absorbent layer. The creases are not so strong due to this, and on removal of the product from its packaging, the product unfolds in its plane better than an absorbent product that does not have a liquid-receiving, flexible foam layer with a bending stiffness that is lower than 0.30 newtons, measured according to the modified Circular Bend Procedure method.

Due to the fact that the crease/creases along the fold/folds in the transverse direction of the product are less strong in the liquid-absorbent, fibrous material layer, this results in the capacity of the liquid-absorbent, fibrous material to spread and distribute the liquid in the desired direction being improved. The problem with material breaks in the liquid-absorbent, fibrous layer along the crease/creases that is/are formed due to the fold/folds in the transverse direction of the product is especially marked for absorbent products with liquid-absorbent fibrous layers that have a high stiffness.

The liquid-receiving layer falls within the invention since the bending stiffness is lower than 0.3 newtons according to the modified Circular Bend Procedure method in any area of the liquid-receiving layer, i.e. in at least one area of the liquid-absorbent layer.

The surface of the liquid-absorbent layer in the plane extension can have local differences in the bending stiffness. It is important, however, that the bending stiffness over any area on the surface of the liquid-absorbent layer is greater than 2.0 newtons, i.e. the liquid-absorbent layer falls within the invention since the bending stiffness is greater than 2.0 newtons according to the modified Circular Bend Procedure method in any area of the liquid-absorbent layer.

According to another embodiment, the surface of the liquid-absorbent layer in the plane extension has at least two different areas with a bending stiffness that is greater than 2.0 newtons according to the modified Circular Bend Procedure method. This means that the surface which has a bending stiffness that is greater than 2.0 newtons according to the modified Circular Bend Procedure method, should be able to be measured on two different areas on the surface of the liquid-absorbent layer, which two areas can be located adjacent to one another or be placed separated from one another.

According to another embodiment, the liquid-absorbent layer has a bending stiffness that is greater than 2.0 newtons according to the modified Circular Bend Procedure method over its entire surface.

According to another embodiment, the liquid-receiving foam layer has a bending stiffness that is lower than 0.25 newtons according to the modified Circular Bend Procedure method. The liquid-receiving layer falls within this embodiment since the bending stiffness is lower than 0.25 newtons according to the modified Circular Bend Procedure method in any area of the liquid-receiving layer, i.e. in at least one area of the liquid-receiving layer.

The liquid-receiving foam layer is a continuous structure. Due to the fact that the foam layer is a continuous structure, it has a good pliability and resilient capacity after it has been exposed to external loading. Fibre and/or filament-based liquid-receiving layers consist of a great number of discrete fibres and/or filaments that are often intermittently joined to one another, but the joining points do not create a continuous structure such as is created by a foamed material, however. Fibre and/or filament-based layers do not have an equally good ability to spring back and regain their original shape following external loading, therefore. Liquid-receiving foam layers have a good capacity for regaining their original shape in all directions following external loading.

The pliability and resilient capacity of the foam also mean that there is a smaller risk of creases arising, or of the layer crumpling, compared with fibre and/or filament-based layers.

The liquid-receiving foam layer is an open-cell continuous structure and acquires a good liquid-receiving capacity in this way.

According to one embodiment, the liquid-absorbent fibrous layer has a bending stiffness that is higher than 3.0 newtons, measured according to the modified Circular Bend Procedure method.

According to one embodiment, each longitudinally running side edge of the liquid-receiving flexible foam layer extends at least along a part of its length outside of each longitudinally running side edge of the liquid-absorbent, fibrous layer. Each longitudinally running side edge of the flexible liquid-receiving foam layer preferably extends outside of each longitudinally running side edge of the liquid-absorbent, fibrous layer along its entire length. The flexible foam material can spring back, i.e. regain substantially its original shape after having been exposed to external loading. The flexible foam material also has a padding effect such that the foam material lines the stiff edges and creates a soft distancing element between the user's skin and the stiff edges of the liquid-absorbent layer.

The liquid-receiving foam layer consists of polyolefin-based foam, polystyrene-based foam, PVC foam, polyvinyl alcohol foam, acrylate foam, for example manufactured according to HIPE technology, polyurethane foam, epoxy foam, latex foam, urea-formaldehyde foam, melamine-formaldehyde foam, silicone foam, viscose foam, carboxymethyl cellulose (CMC) foam, starch form, chitosan foam, alginate foam, polyactide foam, polyglycolide foam and polycaprolactone foam.

It has proved to be an advantage especially for sanitary napkins that are to absorb menstrual liquid, which has a strong colour, that the absorbed liquid that is absorbed into the product is not very visible. Due to the fact that the liquid-receiving layer has an opacity that is greater than 35%, it turned out that the user experiences the product as more pleasing than a product that has liquid-receiving layers that have an opacity that is less than 35%, for example fibre layers of airy, nonwoven material. It is also important that the user finds before using the product that it is a reliable and aesthetically pleasing product. Due to the fact that the liquid-receiving layer has an opacity that is greater than 35%, the difference in size of the absorbent layers in the plane extension is not seen as distinctly, which gives a more reliable impression of the product even before use.

The liquid-receiving flexible foam layer has an absorption capacity that is lower than 0.15 grams of liquid/cm$^3$ of dry test material measured according to the method $CRC_{material layer}$. An advantage of a liquid-receiving foam layer with a low absorption capacity in grams of liquid/cm$^3$ of dry test material is that a smaller area is moistened on the material layer than for a material layer that has a high absorption capacity in grams of liquid/ cm$^3$. A liquid-receiving layer with a low absorption capacity is drained more easily of liquid by an underlying liquid-absorbent layer.

According to another embodiment, each longitudinally running side edge of the liquid-receiving, flexible foam layer extends at least 5.0 millimetres outside of each longitudinally running side edge of the liquid-absorbent fibrous layer. The width of the surface of the liquid-receiving foam material in the longitudinal direction of the product extending outside of the surface of the underlying liquid-absorbent layer should be adapted in such a manner that good flexibility is obtained. It turned out that the width on each longitudinally running side edge of the liquid-receiving layer extending outside of the underlying absorbent layer is preferably between 5-15 mm. As previously described, the flexible foam material has a padding effect such that the foam material lines the stiff edges. In order to obtain a good padding effect it turned out that said distance, 5-15 mm, is optimal. As also described previously, the ability of the foam material to spring back, i.e. to return to substantially its original shape after having been exposed to external loading, is also an important property. The foam material has the ability to regain its original shape in all directions, not only in the direction of the thickness of the material.

According to another embodiment, the total surface of the liquid-receiving flexible foam layer in its plane extension is at least 1.7 times as great as the total surface of the liquid-absorbent, fibrous layer in the plane extension of the product. For some products, for example, for a panty liner that does not need to be able to absorb an equally great volume of menstrual liquid, the total surface of the liquid-absorbent, fibrous layer does not need to be equally great, which means that for such products it can be advantageous if the total surface of the liquid-receiving foam layer in its plane extension is at least 2.0 times as great as the total surface of the liquid-absorbent, fibrous layer in the plane extension of the product. Due to the fact that the open-cell, liquid-receiving foam layer has an opacity that is greater than 35%, the difference in size of the layers is not seen as clearly, which makes the product more aesthetically pleasing and gives the product a more reliable impression. A high opacity of the liquid-receiving layer is especially advantageous, therefore, for products with a large difference of the surface between the liquid-receiving layer and the liquid-absorbent layer.

A small surface of the liquid-absorbent layer can be desirable for certain products, in part for making them thin and discreet and in part for reducing the material cost. Furthermore, an advantage with a large difference on the surface between the liquid-receiving layer and the liquid-absorbent layer is that when the liquid-absorbent layer also functions as a shaping element that follows the body when it moves, a certain difference of the layers can be required for obtaining the desired shaping.

According to an embodiment the absorbent product comprises a liquid-permeable surface material and a liquid-tight backing material, wherein the absorption body is arranged between the liquid-permeable surface material and the liquid-tight backing material, and that the liquid-receiving foam layer is placed against the liquid-permeable surface material and the liquid-absorbent, fibrous layer is placed against the liquid-tight backing material.

However, it is also possible that the absorbent product does not have a separate liquid-permeable surface material. In such an embodiment the liquid-receiving layer is placed closest to the user when the product is being used and thus the surface closest to the user consists of a flexible foam. An advantage of such an embodiment is that the surface closest to the user is soft, smooth and pliable and that the number of layers used in the product is less, which simplifies the manufacturing process and possibly also reduces the material cost.

The invention also relates to folded absorbent products, which have two or more folds in the transverse direction of the product.

The invention also relates to a single-unit packaging containing a folded absorbent product according to one/some of the described embodiments, and a packaging containing a plurality of folded absorbent products according to one/some of the embodiments described. The liquid-receiving flexible foam layer expands relatively quickly from its folded compressed state when the product is removed from its packaging, and in this way the product regains a relatively flat extension relatively quickly, which makes application of the product to the user's briefs easier, and reduces the existing fold lines in the transverse direction of the product. The folded absorbent product in the single-unit packaging thus has a more compressed structure than corresponding absorbent products have in their unfolded state.

In the same way, each of the folded absorbent products in a packaging containing a plurality of folded absorbent products has a more compressed structure than corresponding absorbent products have in their unfolded state.

Upon packing or alternatively before packing of a plurality of folded absorbent products, the folded products are stacked in piles, wherein each pile contains a plurality of products. The stack with a plurality of products is higher before the compression stage than after the compression stage.

SHORT DESCRIPTION OF FIGURES

The invention will be described in greater detail in the following with reference to the exemplary embodiments shown in the enclosed drawings. In the figures:

Figure 1    shows a sanitary napkin viewed from above;

Figure 2    shows a section along line II-II through the sanitary napkin in figure 1

Figure 3    shows an alternative embodiment of the sanitary napkin in figure 1, seen from above

Figure 4    shows a single-unit packaging containing the sanitary napkin in figure 1.

Figure 5    shows a section along line II-II through the single-unit packaging in figure 4

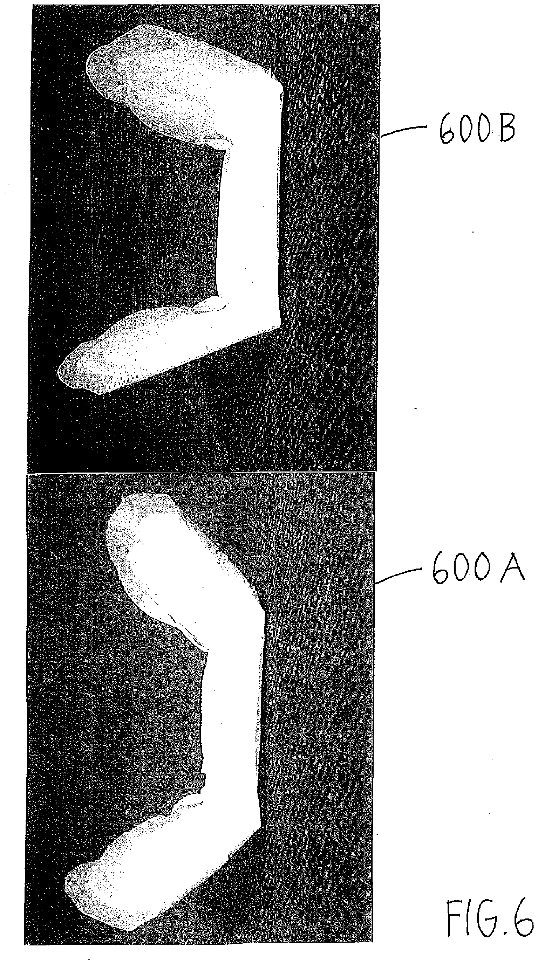

Figure 6    shows photo on sanitary napkins after they have been removed from their packaging. The sanitary napkin 600A is according to the invention and the sanitary napkin 600B is according to the prior art.

DESCRIPTION OF THE EMBODIMENTS:

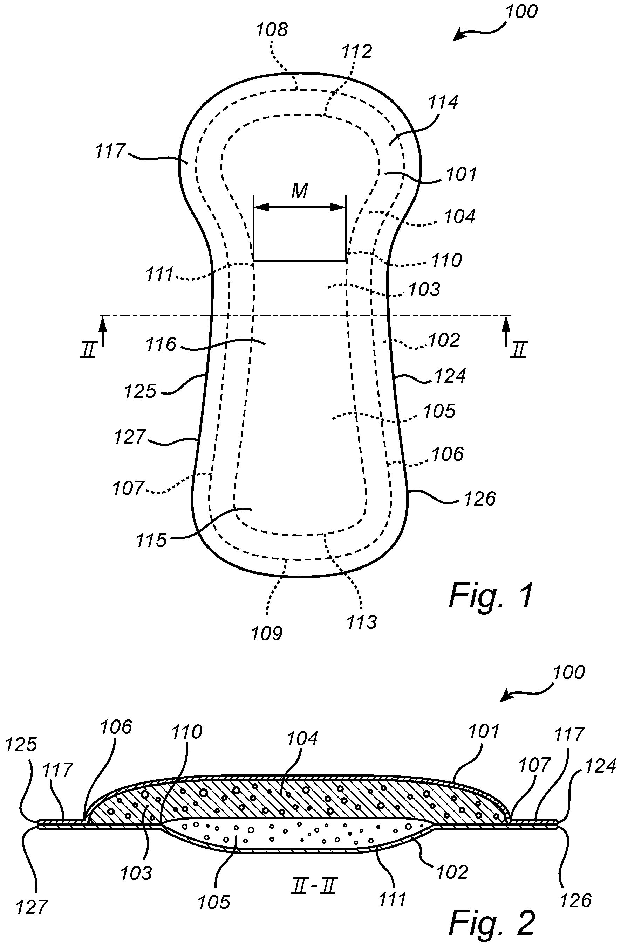

The sanitary napkin 100 shown in figures 1 and 2 is elongated with a longitudinal direction and a transverse direction. The sanitary napkin 100 comprises a liquid-permeable surface material 101 arranged on the top side of the sanitary napkin, i.e. the side of the sanitary napkin intended to be turned toward a user during use, a liquid-tight backing material 102, and an absorption body 103 arranged between the surface material 101 and the backing material 102. The absorption body 103 comprises a flexible, liquid-receiving foam layer 104 placed against the liquid-permeable surface material 101 and a liquid-absorbent fibrous layer 105 placed against the liquid-tight backing material 102, wherein the liquid-receiving foam layer 104 has two opposing, longitudinally running side edges 106, 107 extending in the longitudinal direction and two opposing transverse edges 108, 109 extending in the transverse direction, and the liquid-absorbent, fibrous layer 105 has two opposing longitudinally running side edges 110, 111 extending in the longitudinal direction, and two opposing transverse edges 112, 113 extending in the transverse direction. The liquid-receiving foam layer 104 has a total surface in the plane extension of the product that covers the entire surface of the liquid-absorbent, fibrous layer 105 in its plane extension, and each longitudinally running side edge 106, 107 of the liquid-receiving foam layer 104 extends at least along a part of its length outside of each longitudinally running side edge 110, 111 of the liquid-absorbent, fibrous layer.

The surface material 101 and the backing material 102 have substantially the same plane form as the absorption body 103 but have a somewhat greater extension in the plane, as a result of which they form a projecting edge 117 around the entire periphery of the absorption body 103. Cover layers 102, 103 are mutually connected inside the projecting edge 105, for example by gluing, sewing or welding with heat or ultrasound. It is also possible to design the sanitary towel so that the liquid-receiving layer 104 has the same extension in the plane as the surface material and backing material, wherein both the surface material 101, backing material 102 and also the liquid-receiving layer 104 form a projecting edge 117 along the entire periphery of the liquid-absorbent, fibrous layer 105.

The sanitary napkin in figure 1 is formed with a front part 114, a back part 115 and an intermediate crotch part 116. The front part 114 has a rounded form and is wider than the crotch part 116. It has turned out that all users have a critical area between the groins where the distance between the muscles running down on the inside of the thighs is approximately 30-35 mm.

The width of the sanitary towel in the crotch area is limited in front by said distance between said muscle sinews right in front of the users' groins. Thus, in the transition between the crotch part 116 and the front part 114, the liquid-absorbent layer 105 has a distance M that does not exceed 40 mm and is preferably 30-35 mm. For the sanitary towel 100 in accordance with our invention, it is the width of the liquid-absorbent layer 105 in the transition between the crotch part 116 and the front part 114 that should not be too wide. The reason that it is the width of the liquid-absorbent layer 105 in the crotch part 116 of the product which is most important is that it is this layer that contributes to the product's stiffness. In the direction back from said transition with width M to the end of the crotch part, the width of the liquid-absorbent, fibrous layer 105, which also functions as a stiffening element, can continuously increase to a magnitude of 1.5 times the width M without a risk that the liquid-absorbent layer 105 chafes the user in the crotch. A relatively narrow distance M, at least on the liquid-absorbent layer 105, is also an advantage from the viewpoint of fastening, since a sanitary napkin where the difference in width between the front part 114 and the width of the narrowest part on the crotch part 116 is large, yields a good fastening effect against the user's legs and prevents the sanitary napkin from gliding backwards during use.

The liquid-permeable surface material 101 suitably consists of a conventional liquid-permeable material. Examples of suitable materials are perforated plastic films, non-woven materials, plastic nets or the like.

The liquid-tight backing material 102 is a conventional type and can thus consist of any liquid-tight material suitable for the purpose. Examples of such materials are various types of thin plastic films or nonwoven materials treated to resist the penetration of liquid, for example, by being coated with plastic, wax or the like. Other treatments such as heat calendering for melting a material that was permeable in the beginning to a mainly liquid-tight layer can also be used. Furthermore, the liquid-tight backing material 103 can consist of a liquid-tight surface on absorption body 103. In order to produce an airy product, it is customary to use liquid-tight backing material that is breathable, i.e., has good air permeability. According to the invention the liquid-tight backing material preferably consists of a breathable material. Examples of breathable materials are perforated films, microporous films, macroporous films, nanoporous films, monolithic films, fibrous nonwoven ones and their laminates.

An example of a liquid-absorbent, fibrous layer 105 with a high absorption capacity and a good capacity for transporting liquid is the fibrous material described in WO 94/10953 and WO 94/10956. These materials are present in the form of dry-formed fibre layers with a high density and stiffness and are used directly in an absorbent product without being defibrated at first. The stiffening and absorbent element can also be made from a laminate of several nonwoven layers or tissue layers that are mutually fixed for increased stiffness and that have highly absorbent particles between individual layers. The fixing of the individual layers to each other can take place with binding agents such as adhesive or melted fibres. The highly absorbent particles can also contribute to the bonding. The stiffness is controlled by selecting the number of layers and the amount of binding agent used and by the selection of highly absorbent material and how its adhesive capacity is utilised.

Another example of material in the liquid-absorbent layer is one or more layers of airlaid cellulose layers. This can be prefabricated material which is supplied in roll form for being then cut out in a suitable size, or alternatively the liquid-absorbent layer can be mat-formed and formed in-line during the actual manufacture of the absorbent product.

For example, to obtain compression lines/compression zones, it can be purposeful to have the areas that are compressed at a higher bending stiffness than the surrounding areas. Such a further compression can take place in combination with the compression of the liquid-absorbent layer. Alternatively, a pattern compression can take place in a separate step after the flat compression.

The compression of the liquid-absorbent, fibrous layer can be done in several ways. An example of a common process is one that is called "high-density compression" (HDC), which is described in detail in EP-B-1427658. The compression can also be done in two to three steps that include a preliminary compression and thereafter a compression in one or two steps.

The surface of the liquid-absorbent layer in plane extension can thus exhibit local differences in bending stiffness. However, it is essential that the bending stiffness over one area on the surface of the liquid-absorbent layer is greater than 2.0 newtons, i.e. the liquid-absorbent layer falls within claim 1 if the bending stiffness is greater than 2.0 newtons according to the modified Circular Bend Procedure method in any area of the liquid-absorbent layer.

According to another embodiment the surface of the liquid-absorbent layer in its plane extension comprises at least two different areas with a bending stiffness that is greater than 2.0 newtons according to the modified bending stiffness method. This means that the surface that has a bending stiffness greater than 2.0 newtons according to the modified Circular Bend Procedure method should be able to be measured on two different areas on the surface of the liquid-absorbent layer, which two areas can be adjacent to one another or can be placed at a distance from one another.

The liquid-absorbent layer preferably has a bending stiffness greater than 2.0 newtons according to the modified Circular Bend Procedure method over the entire liquid-absorbent layer. According to this embodiment no area on the liquid-absorbent layer may have a bending stiffness that is lower than 2.0 newtons when measured according to the modified Circular Bend Procedure method.

A suitable liquid-receiving foam layer 104 is polyurethane foam. Polyurethane is a two-component product consisting of polyol and isocyanate that are mixed to polyurethane foam. Polyurethane has either open or closed cells. For use as a liquid-receiving foam layer in an absorbent product, polyurethane foam with open cells is used. Furthermore, polyurethane foam can have different stiffness and for our purpose can be viewed as flexible foams. The liquid-receiving foam is flexible with a low bending stiffness and springs back well, i.e. after being loaded the foam returns to substantially its original form. The liquid-receiving foam layer can consist of thermoplastic foam or thermosetting foams. Examples of useable foams are polyolefin-based foam, polystyrene-based foam, PVC foam, polyvinyl alcohol foam, acrylate foam, for example manufactured according to HIPE technology, polyurethane foam, epoxy foam, latex foam, urea-formaldehyde foam, melamine-formaldehyde foam, silicone foam, viscose foam, carboxymethyl cellulose (CMC) foam, starch foam, chitosan foam, alginate foam, polylactide foam, polyglycolide foam and polycaprolactone foam.

A fastening element in the form of a rectangular area of self-adhesive glue extending in the longitudinal direction is arranged on the outside of the liquid-tight backing layer. When the sanitary napkin 100 is being used, it is placed inside the user's underpants and is fastened in the underpants with the aid of the fastening element. Before use, the fastening element is protected in a conventional manner, for example, by being covered by a protective layer of paper or plastic treated with silicone, or is embossed so that it can be readily separated from the glue when the sanitary towel is to be used. The glue can obviously be alternatively arranged in any pattern suitable for the purpose such as a plurality of longitudinally running strands, an entire covering, arranged in areas only at the front part and/or the back part or the like. Furthermore, other types of fastening elements can be used such as friction covering, press studs, clamps, fastening flaps or the like.

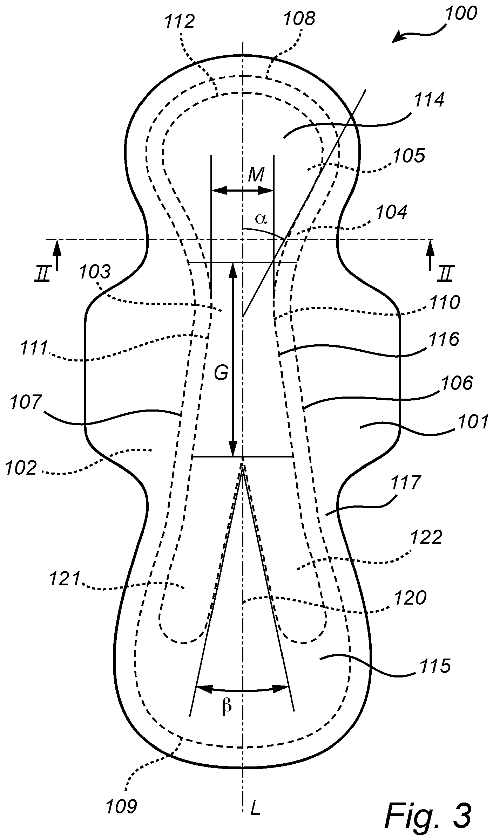

Figure 3 shows an alternative embodiment of the sanitary napkin 100 in accordance with the invention. Figure 3 shows the sanitary napkin from above. The liquid-absorbent, fibrous layer 105 has a shape like a keyhole. The liquid-receiving layer 104 also has a shape like a keyhole but with a greater extension in the longitudinal direction as well as in the transverse direction than the liquid-absorbent, fibrous layer 105. The liquid-absorbent, fibrous layer 105 is also intended to function as a stiffening element and is designed to reduce the risk that the sanitary napkin deforms in an uncontrolled manner. The liquid-absorbent, fibrous layer 105 has a size, shape and stiffness that means that the product retains during the entire time of use a predetermined shape and furthermore is held fast at the intended location on the user.

The liquid-absorbent layer has a width M in the transition between the crotch part 116 and the front part 114, which width is less than 40 mm, preferably 30-35 mm. Both side edges of the front part 114 diverge in a forward direction in the product from said transition M. In this manner the product is prevented from shifting backwards between the user's legs.

In figure 3 there is an angle between a line in the longitudinal direction of the product and each of the side edges of the liquid-absorbent, fibrous layer in the front part designated with $\alpha$. In the case of a large angle $\alpha$, for example in the vicinity of 90°, the edges of the front part can chafe against the user's groins and legs, thus creating discomfort for the user. The smaller the angle $\alpha$ is, the greater the risk that the product slides backwards in between the user's legs. At an angle below 30° this risk is unacceptably great. An angle between 35-45° gives the best balance between a firm hold and comfort.

The sanitary napkin 100 in figure 3 is formed with a crotch length adapted to the user's anatomy. In a sanitary napkin in accordance with the invention, the fact was utilised that most women have a crotch length in the order of 80-100 mm. Therefore, the liquid-absorbent, fibrous layer 105 was created with a corresponding crotch length G in the order of 70-120 mm. Along the crotch, where the user's body shape is substantially plane, the sanitary napkin in accordance with the invention is shaped so as to be relatively stiff laterally, i.e. it is sufficiently stiff so as to not be deformed laterally and form creases. Since it is important that the liquid-absorbent, fibrous layer has a good absorption capacity, it is important to be able to utilise accessible space between the user's legs in the crotch. The width of the sanitary napkin in the crotch area is limited in front by said distance between said muscle sinews right in front of the user's groins. In the direction backwards from said transition area to the end of the crotch part, the width of the liquid-absorbent, fibrous layer 105, which also functions as a stiffening element, can continuously increase to the size of 1.5 times the width M in the transition between crotch part and front part without the risk that the liquid-absorbent layer 105 chafes the user in the crotch.

The liquid-absorbent layer 105 extends slightly over the product's back part 115. The liquid-absorbent layer 105 has a recess 120 in the back part 115 and extending from its end edge and in the direction toward the crotch part 116, by means of which the product can fold along a longitudinally running line in the recess and by means of which the parts, the legs 121 and 122, which are located on both sides of the recess 120, become more flexible than the wider crotch part 116. This recess 120 is for obtaining a good adaptation to and pliability with the body.

The recess 120 is wedge-shaped and symmetrically located relative to the longitudinally running line of symmetry L of the product and forms an angle $\beta$ with a magnitude of 20°. This angle can vary within broad limits but is of course dependent on the shape of the back part 115.

The invention has been described above in connection with a sanitary napkin. It is possible, however, to utilise the invention also for panty liners and incontinence pads.

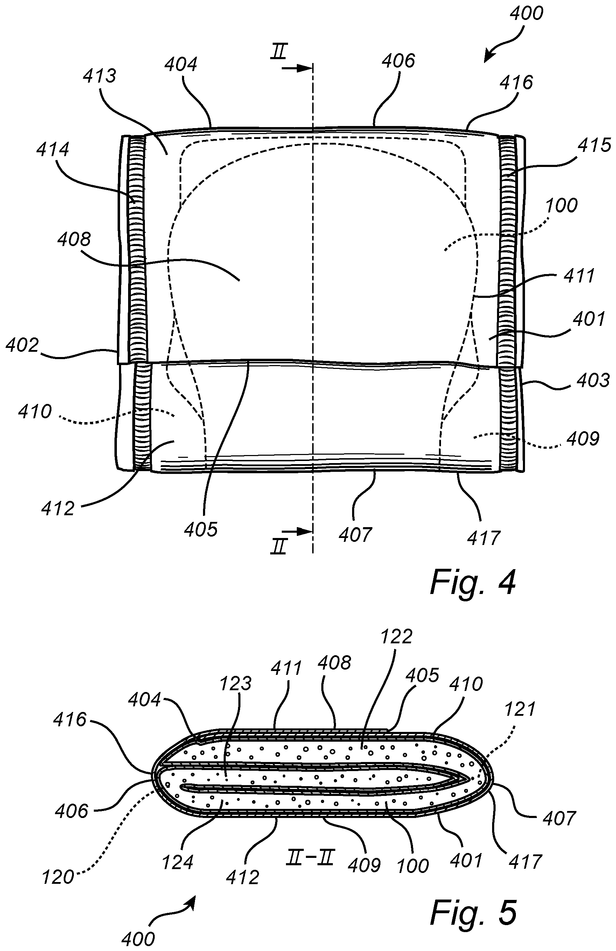

Figure 4 and 5 show a single-unit packaging 400 in which the sanitary napkin 100 is packed. The single-unit packaging 400 is a packaging wrapper and in the description is called packaging wrapper 400. The packaging wrapper 400 is formed by a piece of material 401 with a rectangular shape, wherein the piece of material 401 has two side edges 402, 403, which also form side edges 402, 403 on the packaging wrapper 400, and two end edges 404, 405. Suitable materials for a packaging wrapper 400 according to the invention are thin plastic films, for example polythene films, or polypropylene films. It is of course also possible to use paper or nonwoven material. If the packaging wrapper 400 is also to serve as release material for an adhesive fastening arrangement on the sanitary napkin 100, it is customary for the surface of the packaging wrapper 400 that comes into contact with the fastening arrangement to be treated with release agent in some way. A common release agent treatment is siliconisation.

The packaging wrapper 400 is folded together along a first fold line 406 and a second fold line 407 so that two end panels 408, 410 are formed, and a centre panel 409. The first end panel 408 forms the cover part 411 of the packaging wrapper. The second end panel 410, and the centre panel 409 located between the end panels 408, 410 together form the container part 412 of the packaging wrapper. The container part 412 is closed along the side edges 402, 403 of the packaging wrapper 400 by edge joints 414,415. In the example shown, the edge joints are embossed welds, but it is naturally possible to achieve edge joints in another way, for example with glue. The cover part 411 is also fixed in the edge joints, which means that the packaging wrapper as shown in figure 4 and 5 is in a closed position. It is advantageous if the cover part 411 can easily be detached from the container part along the edge joints 414, 415 without the packaging material being torn apart. This can be achieved by making the edge joints 414, 415 openable at least between the cover part 411 and the container part 412. The edge joints 414, 415 are preferably fully openable, since it is desirable to be able to unwrap the packaging wrapper completely when the sanitary napkin 100 is to be removed from the packaging wrapper 400.

As already discussed, the packaging wrapper 400 has two side edges 402, 403 in its folded and joined form. Furthermore, the packaging wrapper has a first end edge 416 and a second end edge 417, which extend perpendicularly to the side edges 402, 403 and which coincide with the first and second fold line 406, 407 of the packaging wrapper. The first end edge 316 also forms an inner edge on the cover part, while the outer edge 405 of the cover part 411 coincides with the one end edge 405 on the piece of material 401 and is located a little way down on the second end panel 410 on the outside of the container part 412.

Figure 5 shows a section along the line II - II through the packaging wrapper in figure 4, wherein the sanitary napkin 100 has a first fold 120 in the transverse direction of the sanitary napkin along the first fold line 406 of the packaging wrapper, and a second fold 121 in the sanitary napkin's transverse direction along the second fold line 407 of the packaging wrapper, wherein the two end parts 122, 123 of the sanitary napkin 100 and the crotch part 124 of the sanitary napkin are folded against one another so that one end part 122 of the sanitary napkin rests against the second end panel 410 of the packaging wrapper and the crotch part 124 of the sanitary napkin rests against the centre panel 409 of the packaging wrapper and the second end part 123 of the sanitary napkin 100 lies between the first end part 122 and the crotch part 124 of the sanitary napkin 100. The sanitary napkin 100 is composed as previously described of a liquid-permeable surface material 101, a liquid-tight backing material 102 and an absorption body 103 arranged between the surface material and the backing material, wherein the absorption body 103 comprises a liquid-receiving flexible foam layer 104 placed against the liquid-permeable surface material 101 and a liquid-absorbent fibrous layer 105 placed against the liquid-tight backing material 102.

Along the first fold 120 of the sanitary napkin 100 in the transverse direction, which fold coincides with the first fold line 406 of the packaging wrapper, and along the second fold 121 of the sanitary napkin in the transverse direction, which fold coincides with the second fold line 407 of the packaging wrapper, the material layers forming part of the sanitary napkin 100 are pressed together, wherein transversely running fold lines are created in the sanitary towel in the folding process. The size of the fold line, i.e. the degree of compression that is formed on the sanitary napkin in the folding process, is smaller for the sanitary napkin 100 constructed from a liquid-receiving layer of a flexible foam than for a sanitary napkin with a liquid-receiving layer of fibres.

EXAMPLES

The following materials were tested:

1. Polyurethane foam, FXI Foamex Innovations Inc., product code CAZ80A
2. Polyurethane foam, Foamex Innovations Inc., product code C80H2A
3. Polyurethane foam, FoamPartner, Reisgies Schaumstoffe GmbH, Regilen 30WF, item number 190321
4. Polyurethane foam, Caligen Foam Ltd., product code E 50
5. Melamine-based foam, BASF Plastics, Basotect W., product code E 2419 10
6. Polyurethane foam, Woodbridge Foam, product code SM25WH
7. Carded through, air-bound, non-woven, Fiberweb Tenotex, product code Airten 1250W6
8. Cellulose-based, multi-bound airlaid, Glatfelter Falkenhagen GmbH, product code MH080.137
9. Cellulose-based airlaid without SAP, "HDC" manufactured according to EP 1427658B1
10. Cellulose-based airlaid with 25 weight per cent SAP, "HDC" manufactured according to EP 1427658B1
11. Cellulose-based, multi-bound airlaid with 32 weight per cent SAP, Glatfelter Falkenhagen GmbH, product code VF250.103
12. Cellulose-based airlaid without SAP, manufactured according to WO 94/10953 with embossing pattern number 2072

13. Cellulose-based airlaid with 10 weight per cent SAP, manufactured according to WO 94/10953 with embossing pattern "Wave".

Explanations:

SAP = Super-absorbent particles manufactured from crossbonded and partially neutralized acrylic acid

Multi-bond airlaid consists primarily of cellulose fibres that are bound with tex bi-component molten fibres and binding agent of ethylene vinylacetate copolymer

"HDC" = High Density Compression is in the example a highly compressed, defibrated and mat-formed chemical sulphate mass with or without SAP The materials in examples 12 and 13 are based on fling-dried CTMP mass with or without SAP. Materials 1-8 relate to liquid-receiving layers and materials 9-13 relate to liquid-absorbent layers. In the liquid-receiving layers 1-8, materials 1-6 relate to liquid-receiving, open-cell foam layers and materials 7 and 8 relate to liquid-receiving, fibrous layers. In the liquid-absorbent layers 9-13 all relate to liquid-absorbent, fibrous layers.

Density measurement

The measurements were carried out in accordance with the EDANA method WSP 130.1.

In order to measure the average density of a material layer in an absorbent product it is important that the various material layers included in the absorbent product are separated with caution. The material specimen to be measured is clipped out of the material layer. A loading pressure of 0.5 kPa is applied to the material specimen by a foot with an area of 45x45 mm and that is less than the area of the material specimen. Then the thickness of the material specimen is measured. The surface weight of the material sample, i.e. $gram/cm^2$, is weighed and calculated forward. Then, the average density of the material specimen is calculated by dividing the surface weight by the thickness.

Result:

| Material | Thickness | Density (kg/m$^3$) |
| --- | --- | --- |
| 1 | 2.0 | 31 |
| 2 | 2.0 | 29 |
| 3 | 2.0 | 29 |
| 4 | 2.8 | 31 |
| 5 | 1.7 | 10 |
| 6 | 2.1 | 23 |
| 7 | 1.6 | 30 |
| 8 | 1.3 | 65 |
| 9 | 2.5 | 205 |
| 10 | 1.6 | 300 |
| 11 | 1.4 | 216 |
| 12 | 1.0 | 350 |
| 13 | 0.8 | 330 |

Method description for modified Circular Bend Procedure

The method for the modified Circular Bend Procedure was carried out according to a modified version of ASTM D 4032-82 (Circular Bend Procedure). The method for the modified Circular Bend Procedure is described in detail in EP 336 578. The measuring was carried out in the same manner as described in EP 336 578.

The apparatus that was used is the Instron Model No 5965. Instron Model No 5965 is manufactured by Instron Engineering Corporation. The bending resistance in the material sample was measured by measuring the maximum bending stiffness. According to the method, the maximum bending stiffness is constituted by a simultaneous deformation in several directions of a material sample, wherein one of the surfaces of the material sample becomes concave and the other surface of the material sample becomes convex. The method for the modified Circular Bend Procedure supplies a force value that is the bending resistance, i.e. simultaneous medium stiffness in all directions.

The equipment used for the method of the modified Circular Bend Procedure is a modified Circular Bend Stiffness Tester that has the following parts:

A smooth-polished steel plate with measurements 102.0 x 102.0 x 6.35 mm that has a circular opening with a diameter of 18.75 mm. The circular opening has a bevelled edge that is 45 degrees to a depth of 4.75 mm. A pressure rod with a total length of 72.2 mm, a diameter of 6.25 mm and a spherical end with a radius of 2.97 mm was used. A sharp needle tip projects 0.88 mm out from the spherical end. The pressure rod was mounted concentrically and has the same magnitude of play in all directions. The end of the pressure rod is placed well over the plate with the circular opening. From this position the downwardly directed strike of the spherical tip is so long that it precisely extends to the bottom of the circular opening of the plate. However, an indication of the distance of the needle tip (0.88 mm) is not included. A tester of draught and compression with a loading cell adapted for Instron Model No. 5965 was used.

Production of test materials and calculation of the average value:

The liquid-receiving- respectively the liquid-absorbent material layer from five absorbent products was measured and then the average value was calculated.

In those cases where the liquid-absorbent layer comprises areas with different bending stiffness, for example, an area that is more compressed than an adjacent area, the liquid-absorbent layer falls within the scope of protection, since any area has a bending stiffness that is greater than 2.0 newtons.

In those cases where the liquid-receiving foam layer comprises areas with different bending stiffness, for example, an area that is more compressed than an adjacent area, the liquid-receiving foam layer falls in a corresponding manner within the scope of protection, since any area has a bending stiffness that is lower than that which is indicated in the claim.

When measuring material layers in an absorbent product, it is essential that the liquid-receiving foam layer is separated from the liquid-absorbent layer with great care so that the material layers do not break into pieces during the separating.

Implementation:

The implementation was made exactly in accordance with the description in EP 0 336 578 A1. The material specimens are stamped out or cut out and have an area of 37.5 x 37.5 mm. The material specimens were conditioned for two hours at a temperature of $21 \pm 1$ °C and a relative air humidity of $50 \pm 2\%$. The pressure rod should be moved downward at a rate of 50.0 cm/min.

The material specimen is then placed centred over the opening of the steel plate. The surface of the material specimen that is turned in the product towards the liquid-permeable surface material was turned during the taking of the specimen towards the pressure rod and the surface of the material sample that was turned in the product towards the liquid-impervious backing material was turned during the taking of the specimen towards the steel plate. The pressure plate was then put in motion and the maximum force was measured. The value of the maximum force was rounded off to the nearest gram.

Result:

| Material | Bending stiffness (newtons) |
| --- | --- |
| 1 | 0.29 |
| 2 | 0.16 |
| 3 | 0.20 |
| 4 | 0.10 |
| 5 | 0.27 |
| 6 | 0.18 |
| 7 | 0.07 |
| 8 | 0.59 |
| 9 | 3.43 |
| 10 | 2.28 |
| 11 | 3.40 |
| 12 | 6.55 |
| 13 | 2.94 |

Materials 1-8 are liquid-receiving layers. The result shows that layers 1-6 that are open-cell foam materials and also material 7, that is a carded through air-bound nonwoven material, have a bending stiffness that is lower than 0.30 newtons. In contrast thereto, material 8, which is a cellulose-based, multi-bound airlaid, exhibits a bending stiffness that is greater than 0.30 newtons. Materials 9-13 are liquid-absorbent layers that exhibit a bending stiffness greater than 2.0 newtons.

Measurement of absorption capacity according to CRC$_{material layer}$

CRC$_{matenal layer}$ is based on WSP 241.3, which is a standard method for superabsorbent polyacrylate in powder or granular form. WSP 241.3 is modified according to the following with the aim of adapting the method to sheet- and mat-shaped material with a thickness of 0.5 mm - 4.0 mm measured under a load that is 0.5 kPa.
The following modifications were made:

Nonwoven bag: according to WSP 241.3, the nonwoven bag has a size that is between 60x40 mm$^2$ and 60-85 mm$^2$. We measured using a nonwoven bag that is 90 x 90 mm$^2$. According to the method description for WSP 241.3, the size of the nonwoven bag is indicated in para. 3 "Terms and definitions" in para. 6.1 "Apparatus" and in para. 8.1 "Procedure".

Analytical balance: according to WSP 241.3, the analytical balance must be able to weigha mass of between 0.180 and 0.220 grams. According to the measurement we performed, the analytical balance must be able to weigh a mass of between 0.200 and 3000 grams.

Centrifuging was carried out according to WSP 241.3, wherein the acceleration was 95 g and an inner diameter of "basket or rotor mesh" that is 235 mm was used.
Test specimen: according to WSP 241.3, between 0.180 grams and 0.220 grams of superabsorbent is weighed and placed in a teabag (nonwoven bag), see para. 8.3 Procedure. On measuring according to the invention, the stamped out, circular test material with a diameter that is 80 mm was weighed and placed in the teabag (nonwoven bag).
According to WSP 241.3, see para. 8.4, the dry polymer is weighed. On measuring according to the invention, the stamped out, circular test material is weighed.
According to WSP 241.3, see para. 8.8, at least 1 litre of saline solution is used for 10 teabags (nonwoven bags). On measuring according to the invention, at least 1 litre of saline solution is used for 4 teabags (nonwoven bags).
According to WSP 241.3, see para. 8.13 and 8.14, the test specimen will absorb the saline solution for 30 minutes. On measuring according to the invention, the test specimen will absorb the saline solution for 10 minutes.
According to WSP 241.3, see para. 8.17, the centrifuge is set to obtain a centrifugal acceleration of 250 g. On measuring according to the invention, the centrifuge is set to obtain a centrifugal acceleration of 95 g.
According to WSP 241.3, see para. 8.22 and 8.23, "PA superabsorbent gel" is written. On measuring according to the invention, the stamped out, circular test material is referred to.
According to WSP 241.3, see para. 9.10, "polymer-based absorbent materials" is written. On measuring according to the invention, the stamped out, circular test material is referred to.
According to WSP 241.3, see para. 9.11, gram per gram (g.g$^{-1}$) is written. On measuring according to the invention, gram/cm$^3_{dry material}$ is referred to.

Results:

| Material | Absorption capacity (grams liquid/ cm$^3_{dry material}$) |
|---|---|
| 1 | 0.14 |
| 2 | 0.12 |
| 3 | 0.06 |
| 4 | 0.05 |
| 5 | 0.13 |
| 6 | 0.09 |
| 7 | 0.04 |
| 8 | 0.30 |
| 9 | 0.91 |
| 10 | 3.10 |
| 11 | 1.66 |
| 12 | 1.40 |

(continued)

| Material | Absorption capacity (grams liquid/ $cm^3_{dry\ material}$) |
|---|---|
| 13 | 2.18 |

An absorption capacity lower than 0.15 grams liquid/ $cm^3_{dry\ material}$ is preferred for the liquid-receiving layer. $CRC_{matenal\ layer}$ is a method that measures the absorption capacity in grams liquid/ $cm^3_{dry\ material}$, and is a measure of how easily a material layer is drained of liquid. A low value of $CRC_{matenal\ layer}$, i.e. a low absorption capacity in grams liquid/ $cm^3_{dry\ material}$, is obtained for liquid-receiving layers with good drainage. A high value of $CRC_{matenal\ layer}$, i.e. a high absorption capacity in grams liquid/ $cm^3_{dry\ material}$, is obtained for liquid-receiving layers that have low drainage.

One reason for measuring the absorption capacity in grams liquid/ $cm^3$dry material instead of measuring the absorption capacity in grams liquid/ gram dry material, i.e. in a unit of volume instead of a unit of weight, is that a thin material is desirable to obtain a comfortable and discreet product, so the thickness of the liquid-receiving layer is therefore more relevant than the weight of the liquid-receiving layer,. For a thin material to be able to acquire a good liquid-receiving capacity, partly in the event of a first wetting, but also upon repeated wetting, it is advantageous to have an open structure with large pores, which means a structure with a low density. A material with low density can receive a larger quantity of liquid per unit of volume ($cm^3$) than a material with higher density. Furthermore, the good liquid-receiving capacity is retained even with repeated wetting for liquid-receiving layers that effectively drain the liquid to underlying absorption layers, in spite of the fact that the material is thin.

When the drainage is good, i.e. a low absorption capacity in grams liquid/ $cm^3$, a smaller area of the material layer becomes wet than for a material layer that has low drainage, i.e. a high absorption capacity in grams liquid/ $cm^3$. Another advantage of liquid-receiving open-cell foam layers with a low absorption capacity in grams liquid/ $cm^3$ is that the surface of the material layer is drier after wetting than a material layer with a higher absorption capacity.

Opacity

The measurements were carried out in accordance with EDANA's method WSP 60.1.

Opacity is a concept and a magnitude in optics that is used to indicate transparency, that is, translucency. Opacity measures the degree of impenetrability for radiation through a material or transmission medium. Material that entirely lacks the ability to let light through is called opaque. The opacity was measured with a spectrophotometer with measurement in the YXY colour system (CIE 1931). The spectrophotometer used was of the brand Minolta Chroma Meter CR 300 (the y value was used) (CIE Illuminant D65). Calibration plate No. 16133079 was used as white standard background with a reflectance of 0.89. Black velvet was used as black standard background with a reflectance of 0.005.

The implementation was made in accordance with EDANA's method WSP 60.1.

The opacity (contrast ratio $C_0$.89) is calculated as follows:

$$\text{Contrast ratio } C_0.89 = R_b/R_w \times 100$$

$R_b$ = brightness, a specimen piece against black standard background
$R_w$ = brightness, a specimen piece against white standard background

The results are indicated with accuracy in whole numbers.

Result:

| Material | Opacity (contrast ratio $C_0$.89, %) |
|---|---|
| 1 | 57 |
| 2 | 37 |
| 3 | 39 |
| 5 | 48 |
| 6 | 37 |

**Claims**

**1.** A folded absorbent product (100) having at least one fold in the transverse direction of the product, wherein the absorbent product comprises an absorption body (103), wherein the absorbent product is a sanitary napkin, a panty liner or a incontinence pad, **characterised by that** the absorption body (103) comprises in its thickness direction a liquid-receiving, flexible foam layer (104) and a liquid-absorbent fibrous layer (105), wherein the liquid-receiving flexible foam layer (104) has a total surface in the plane extension of the product that at least covers the entire surface of the liquid-absorbent, fibrous layer (105) in the plane extension, wherein the liquid-receiving, flexible foam layer (104) consists of polyolefin-based foam, polystyrene-based foam, PVC foam, polyvinyl alcohol foam, acrylate foam, polyurethane foam, epoxy foam, latex foam, urea-formaldehyde foam, melamine-formaldehyde foam, silicone foam, viscose foam, carboxymethyl cellulose (CMC) foam, starch foam, chitosan foam, alginate foam, polyactide foam, polyglycolide foam or polycaprolactone foam, and has a bending stiffness that is less than 0.30 newtons measured according to the modified Circular Bend Procedure method and that the liquid-absorbent, fibrous layer (105) has a bending stiffness greater than 2.0 newtons measured according to the modified Circular Bend Procedure method, and wherein the liquid-receiving, flexible foam layer (104) has an absorption capacity that is lower than 0.15 grams liquid/cm$^3$ of dry test material measured according to the method $CRC_{material\ layer}$.

2. A folded absorbent product (100) according to claim 1, wherein the liquid-receiving, flexible foam layer (104) consists of a continuous open-cell structure.

3. A folded absorbent product according to claim 1 or claim 2, wherein the liquid-absorbent, fibrous layer (105) has a bending stiffness that is greater than 3.0 newtons measured according to the modified Circular Bend Procedure method.

4. A folded absorbent product according to any one of claims 1-3, wherein each of the longitudinally running side edges (106, 107) of the liquid-receiving, flexible foam layer (104) extends at least along a part of its length outside of each of the longitudinally running side edges (110, 111) of the liquid-absorbent, fibrous layer (105).

5. A folded absorbent product according to claim 4, wherein each longitudinally running side edge (106, 107) of the liquid-receiving, flexible foam layer (104) extends outside of each longitudinally running side edge (110, 111) of the liquid-absorbent, fibrous layer (105) along its entire length.

6. A folded absorbent product according to any one of the previous claims, wherein the liquid-receiving, flexible foam layer (104) has an opacity that is greater than 35%.

7. A folded absorbent product according to any one of the previous claims, wherein each longitudinally running side edge (106, 107) of the liquid-receiving, flexible foam layer (104) extends at least 5.0 millimetres outside of each longitudinally running side edge (110, 111) of the liquid-absorbent, fibrous layer (105).

8. An absorbent product according to any one of the previous claims, wherein the total surface of the liquid-receiving, flexible foam layer (104) in the plane extension is at least 1.7 times as great as the total surface of the liquid-absorbent, fibrous layer (105) in the plane extension of the product.

9. An absorbent product according to any one of the previous claims, wherein the product comprises a liquid-permeable surface material (101) and a liquid-tight backing material (102), wherein the absorption body (103) is arranged between the liquid-permeable surface material (101) and the liquid-tight backing material (102), and that the flexible, liquid-receiving foam layer (104) is placed against the liquid-permeable surface material (101) and the liquid-absorbent, fibrous layer (105) is placed against the liquid-tight backing material (102).

10. A folded absorbent product according to any one of the previous claims contained in a single-unit packaging.

11. A folded absorbent product according to claim 10, wherein the folded absorbent product in the single-unit packaging has a more compressed structure than corresponding absorbent products in their unfolded state.

12. Plurality of folded absorbent products according to any one of the previous claims 1-9 contained in a package.

13. Plurality of folded absorbent products according to claim 12, wherein each of the folded absorbent products in the packaging has a more compressed structure than corresponding absorbent products in their unfolded state.

14. Plurality of folded and compressed absorbent products according to any one of claims 1-9 comprised in a pile, wherein the length of the pile is greater before the compression stage than after the compression stage.

**Patentansprüche**

1. Gefaltetes saugfähiges Produkt (100) mit mindestens einer Faltung in der Querrichtung des Produkts, wobei das saugfähige Produkt einen saugfähigen Körper (103) aufweist, und das saugfähige Produkt eine Damenbinde, ein Pantyliner oder ein Inkontinenzpad ist, **dadurch gekennzeichnet, dass** der saugfähige Körper (103) in seiner Dickenrichtung eine flüssigkeitsaufnehmende, flexible Schaumlage (104) und eine flüssigkeitsabsorbierende Faserlage (105) aufweist, die flüssigkeitsaufnehmende, flexible Schaumlage (104) eine Gesamtfläche in der ebenen Ausdehnung des Produkts aufweist, die mindestens die Gesamtfläche der flüssigkeitsabsorbierenden Faserlage (105) in der ebenen Ausdehnung aufweist, die flüssigkeitsaufnehmende, flexible Schaumlage (104) aus Schaum auf Polyolefinbasis, Schaum auf Polystyrolbasis, PVC-Schaum, Polyvinylalkoholschaum, Acrylatschaum, Polyurethanschaum, Epoxidschaum, Latexschaum, Harnstoff-Formaldehyd-Schaum, Melaminformaldehydschaum, Silikonschaum, Viskoseschaum, Carboxymethylcellulose-Schaum (CMC-Schaum), Stärkeschaum, Chitosanschaum, Alginatschaum, Polylactidschaum, Polyglycolidschaum oder Polycaprolactonschaum besteht und eine nach der modifizierten Circular-Bend-Procedure-Methode gemessene Biegesteifigkeit aufweist, die geringer als 0,3 N ist, und dass die flüssigkeitsabsorbierende Faserlage (105) eine nach der modifizierten Circular-Bend-Procedure-Methode gemessene Biegesteifigkeit aufweist, die größer als 2,0 N ist, und wobei die flüssigkeitsaufnehmende, flexible Schaumlage (104) eine nach der $CRC_{material\ layer}$-Methode gemessene Absorptionskapazität aufweist, die geringer als 0,15 g Flüssigkeit/cm$^3$ trockenen Testmaterials ist.

2. Gefaltetes saugfähiges Produkt (100) nach Anspruch 1, bei dem die flüssigkeitsaufnehmende, flexible Schaumlage (104) aus einer durchgehenden, offenzelligen Struktur besteht.

3. Gefaltetes saugfähiges Produkt nach Anspruch 1 oder 2, bei dem die flüssigkeitsabsorbierende Faserlage (105) eine nach der modifizierten Circular-Bend-Procedure-Methode gemessene Biegesteifigkeit aufweist, die größer als 3,0 N ist.

4. Gefaltetes saugfähiges Produkt nach einem der Ansprüche 1-3, bei dem sich jede der in Längsrichtung verlaufenden Seitenkanten (106, 107) der flüssigkeitsaufnehmenden, flexiblen Schaumlage (104) zumindest entlang eines Teils ihrer Länge außerhalb jeder der in Längsrichtung verlaufenden Seitenkanten (110, 111) der flüssigkeitsabsorbierenden Faserlage (105) erstreckt.

5. Gefaltetes saugfähiges Produkt nach Anspruch 4, bei dem sich jede in Längsrichtung verlaufende Seitenkante (106, 107) der flüssigkeitsaufnehmenden, flexiblen Schaumlage (104) entlang ihrer gesamten Länge außerhalb jeder in Längsrichtung verlaufenden Seitenkante (110, 111) der flüssigkeitsabsorbierenden Faserlage (105) erstreckt.

6. Gefaltetes saugfähiges Produkt nach einem der vorstehenden Ansprüche, bei dem die flüssigkeitsaufnehmende, flexible Schaumlage (104) eine Opazität aufweist, die größer als 35% ist.

7. Gefaltetes saugfähiges Produkt nach einem der vorstehenden Ansprüche, bei dem sich jede in Längsrichtung verlaufende Seitenkante (106, 107) der flüssigkeitsaufnehmenden, flexiblen Schaumlage (104) mindestens 5,0 mm außerhalb jeder in Längsrichtung verlaufenden Seitenkante (110, 111) der flüssigkeitsabsorbierenden Faserlage (105) erstreckt.

8. Saugfähiges Produkt nach einem der vorstehenden Ansprüche, bei dem die Gesamtfläche der flüssigkeitsaufnehmenden, flexiblen Schaumlage (104) in der ebenen Ausdehnung mindestens 1,7-mal größer ist als die Gesamtfläche der flüssigkeitsabsorbierenden Faserlage (105) in der ebenen Ausdehnung des Produkts.

9. Saugfähiges Produkt nach einem der vorstehenden Ansprüche, wobei das Produkt ein flüssigkeitsdurchlässiges Flächenmaterial (101) und ein flüssigkeitsundurchlässiges Stützmaterial (102) aufweist, wobei der saugfähige Körper (103) zwischen dem flüssigkeitsdurchlässigen Flächenmaterial (101) und dem flüssigkeitsundurchlässigen Stützmaterial (102) angeordnet ist und die flexible, flüssigkeitsaufnehmende Schaumlage (104) an dem flüssigkeitsdurchlässigen Flächenmaterial (101) platziert ist und die flüssigkeitsabsorbierende Faserlage (105) an dem flüssigkeitsundurchlässigen Stützmaterial (102) platziert ist.

10. Gefaltetes saugfähiges Produkt nach einem der vorstehenden Ansprüche, das in einer Einzelverpackung enthalten ist.

11. Gefaltetes saugfähiges Produkt nach Anspruch 10, wobei das gefaltete saugfähige Produkt in der Einzelverpackung eine stärker komprimierte Struktur aufweist als entsprechende saugfähige Produkte in ihrem entfalteten Zustand.

**12.** Vielzahl gefaltete saugfähige Produkte nach einem der vorstehenden Ansprüche 1-9, die in einer Verpackung enthalten sind.

**13.** Vielzahl gefalteter, saugfähiger Produkte nach Anspruch 12, bei der jedes der gefalteten saugfähigen Produkte in der Verpackung eine stärker komprimierte Struktur aufweist als entsprechende saugfähige Produkte in ihrem entfalteten Zustand.

**14.** Vielzahl gefalteter und komprimierter, saugfähiger Produkte nach einem der Ansprüche 1-9, die zu einem Stapel gehören, wobei die Länge des Stapels vor der Kompressionsstufe größer ist als nach der Kompressionsstufe.

**Revendications**

**1.** Produit absorbant plié (100) ayant au moins un pli dans le sens transversal du produit, le produit absorbant comprenant un corps d'absorption (103), le produit absorbant étant une serviette hygiénique, un protège-slip ou une serviette pour incontinent, **caractérisé en ce que** le corps d'absorption (103) comprend dans le sens de l'épaisseur une couche de mousse souple recevant les liquides (104) et une couche fibreuse absorbant les liquides (105), la couche de mousse souple recevant les liquides (104) ayant une surface totale dans l'extension plane du produit qui recouvre au moins la totalité de la surface de la couche fibreuse absorbant les liquides (105) dans l'extension plane, la couche de mousse souple recevant les liquides (104) étant constituée de mousse à base de polyoléfine, de mousse à base de polystyrène, de mousse de PVC, de mousse de poly(alcool vinylique), de mousse d'acrylate, de mousse de polyuréthane, de mousse d'époxyde, de mousse de latex, de mousse d'urée-formaldéhyde, de mousse de mélamine-formaldéhyde, de mousse de silicone, de mousse de viscose, de mousse de carboxyméthylcellulose (CMC), de mousse d'amidon, de mousse de chitosane, de mousse d'alginate, de mousse de polylactide, de mousse de polyglycolide ou de mousse de polycaprolactone et ayant une rigidité en flexion mesurée selon la méthode de la procédure de flexion circulaire modifiée qui est inférieure à 0,30 newton et **en ce que** la couche fibreuse absorbant les liquides (105) a une rigidité en flexion mesurée selon la méthode de la procédure de flexion circulaire modifiée supérieure à 2,0 newtons, et dans lequel la couche de mousse souple recevant les liquides (104) a une capacité d'absorption mesurée selon la méthode $CRC_{couche\ de\ matériau}$ qui est inférieure à 0,15 gramme de liquide/cm$^3$ de matériau testé sec.

**2.** Produit absorbant plié (100) selon la revendication 1, dans lequel la couche de mousse souple recevant les liquides (104) est constituée d'une structure continue à alvéoles ouverts.

**3.** Produit absorbant plié selon la revendication 1 ou la revendication 2, dans lequel la couche fibreuse absorbant les liquides (105) a une rigidité en flexion mesurée selon la méthode de la procédure de flexion circulaire modifiée qui est supérieure à 3,0 newtons.

**4.** Produit absorbant plié selon l'une quelconque des revendications 1-3, dans lequel chacun des bords latéraux courant longitudinalement (106, 107) de la couche de mousse souple recevant les liquides (104) s'étend au moins le long d'une partie de sa longueur à l'extérieur de chacun des bords latéraux courant longitudinalement (110, 111) de la couche fibreuse absorbant les liquides (105).

**5.** Produit absorbant plié selon la revendication 4, dans lequel chaque bord latéral courant longitudinalement (106, 107) de la couche de mousse souple recevant les liquides (104) s'étend à l'extérieur de chaque bord latéral courant longitudinalement (110, 111) de la couche fibreuse absorbant les liquides (105) le long de toute sa longueur.

**6.** Produit absorbant plié selon l'une quelconque des revendications précédentes, dans lequel la couche de mousse souple recevant les liquides (104) a une opacité qui est supérieure à 35 %.

**7.** Produit absorbant plié selon l'une quelconque des revendications précédentes, dans lequel chaque bord latéral courant longitudinalement (106, 107) de la couche de mousse souple recevant les liquides (104) s'étend au moins sur 5,0 millimètres à l'extérieur de chaque bord latéral courant longitudinalement (110, 111) de la couche fibreuse absorbant les liquides (105).

**8.** Produit absorbant selon l'une quelconque des revendications précédentes, dans lequel la surface totale de la couche de mousse souple recevant les liquides (104) dans l'extension plane est au moins 1,7 fois plus grande que la surface totale de la couche fibreuse absorbant les liquides (105) dans l'extension plane du produit.

9. Produit absorbant selon l'une quelconque des revendications précédentes, le produit comprenant une matière de surface perméable aux liquides (101) et une matière d'envers étanche aux liquides (102), dans lequel le corps d'absorption (103) est disposé entre la matière de surface perméable aux liquides (101) et la matière d'envers étanche aux liquides (102) et la couche de mousse souple recevant les liquides (104) est placée contre la matière de surface perméable aux liquides (101) et la couche fibreuse absorbant les liquides (105) est placée contre la matière d'envers étanche aux liquides (102).

10. Produit absorbant plié selon l'une quelconque des revendications précédentes contenu dans un emballage individuel.

11. Produit absorbant plié selon la revendication 10, le produit absorbant plié dans l'emballage individuel ayant une structure plus comprimée que les produits absorbants correspondants dans leur état déplié.

12. Pluralité de produits absorbants pliés selon l'une quelconque des revendications 1-9 précédentes contenus dans un emballage.

13. Pluralité de produits absorbants pliés selon la revendication 12, chacun des produits absorbants pliés dans l'emballage ayant une structure plus comprimée que les produits absorbants correspondants dans leur état déplié.

14. Pluralité de produits absorbants pliés et comprimés selon l'une quelconque des revendications 1-9 compris dans une pile, la longueur de la pile étant plus grande avant l'étape de compression qu'après l'étape de compression.

*Fig. 1*

*Fig. 2*

*Fig. 3*

**Fig. 4**

**Fig. 5**

600 B

600 A

FIG.6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010178932 A
- US 20050131371 A1
- US 20080103468 A1
- WO 9410953 A
- WO 9410956 A
- EP 1427658 B
- EP 1427658 B1
- EP 336578 A
- EP 0336578 A1